# EUROPEAN PATENT APPLICATION

(11) **EP 1 174 816 A2**
(43) Date of publication of application: **23.01.2002**
(21) Application number: 01303871.6
(22) Date of filing: 27.04.2001
(51) Int. Cl.: G06F 19/00

(54) **Method and system for managing chronic disease and wellness online**

(30) Priority: 28.04.2000 US 200556 P
(71) Applicant: Chan, Bryan K., Redwood City, CA 94061 (US); Chu, Lawrence F., Redwood City, CA 94061 (US)
(72) Inventor: Chan, Bryan K., Redwood City, CA 94061 (US); Chu, Lawrence F., Redwood City, CA 94061 (US)
(74) Representative: Perkins, Sarah

(57) **Abstract**

A method and system (100) for managing a multi-domain health and wellness program using remotely located terminal devices (108, 110, 124), software agents and remotely stored (146) participant information associated with definable access levels. The system referred to herein as Intelligent Health Management Technology (IHMT) system, provides assistance in regards to the management of chronic diseases in conjunction with multi-domain health and wellness programs. The system collects personal health information and medical record data and analyzes the information, and simulates medical decision-making process and is based on general medical decision making principles, common sense principles, and specific logic for a given IHMT module. As a result, customized recommendations are provided and may include computer generated recommendations and input from participating third parties (i.e., doctors, dieticians, pharmacists etc.).

## Description

The present invention generally relates to the area of healthcare systems and particularly relates to methods and systems for providing over communication networks, medical recommendations related to a medical condition based on latest inputs from a user and historic data about the user in conjunction with proven, widely accepted and standards of clinical and decision making analysis, wherein the communication networks may include the Intranet, the Internet and a wireless data network and the medical condition may include a chronic disease. More particularly, the present invention relates to a method and system for managing, updating and selectively accessing data associated with various medical conditions while at the same time allowing selective access to the data for the purpose of promoting the health and wellbeing of individuals or groups accessing the data.

Over 90 million people suffer from chronic medical conditions like diabetes, asthma, and heart disease. Chronic illnesses account for approximately 75% of the total healthcare costs in the United States, that's 75% of $1.25 Trillion. Diabetes alone affects approximately 1 in 17 Americans, results in almost $100 Billion dollars in spending, and accounts for more than 15% of pharmaceutical sales. The prevalence of other chronic diseases is large as well ― for instance, asthma affects more than 14 million Americans.

Studies have shown that close monitoring and management may be beneficial to patients with lifelong chronic illnesses. Regular management of disease may help to identify complications of disease before they become severe. For example, daily monitoring of blood sugar levels is crucial to the health of patients with diabetes. Chronically high levels of blood sugars may cause patients to be at risk for developing costly complications such as diabetic retinopathy, neuropathies, and renal diseases. Acute elevations of blood sugar levels may lead to life-threatening medical emergencies, such as diabetic ketoacidosis, which requires a costly hospitalization, often in the intensive care unit. Such emergencies could be prevented with improved management of the chronic disease. Similarly, it is clear that a healthy lifestyle in terms of diet, exercise, and other habits plays an important role in disease prevention and minimization. For example, patients who are obese and have a body-mass index exceeding the norm may be at risk for developing chronic diseases such as diabetes. Likewise, patients who are unable to maintain a low salt diet of may develop more severe high blood pressure (e.g., hypertension).

Numerous strategies for disease management have been designed and implemented to identify clinical findings that predict the need for "stitch-in-time" preventive interventions at certain stages of these disorders. Disease management is in-context prevention, and aims at health risk avoidance. Preventive measures are derived from analysis of large pools of data. The data is typically acquired through the diligent and collective efforts of caregivers, family members and patients. However, the pools of data are less effective unless the data is closely related to those who require access to the data in a timely fashion and a medical judgement could be reliably derived from the data. For example, timely access to the historic data about diabetes and a diabetic patient can facilitate pre-emptive medical care for the diabetic patient, effective health and wellness programs and peace of mind for the patient and his/her family.

There is therefore a great need for a health management system that can facilitate and improve the management of chronic disease and maintenance of wellness and thus help to identify and prevent worsening health. Further there is another need for an easy and secure access to the health management system and patient medical records from anywhere at anytime.

According to one aspect of the present invention, a system, referred to herein as Intelligent Health Management Technology (IHMT) system, is configured to facilitate and improve the management of chronic diseases in conjunction with multi-domain health and wellness programs. The system collects personal health information and medical record data and analyzes the information, and makes physician-like recommendations based on the available data where the recommendations may include computer generated recommendations and input from participating third parties (i.e., doctors, dieticians, pharmacists etc.). More specifically, the present invention utilizes intelligent agents, network based software application modules having definable access levels, digital credentials, access rules and personalized contact lists to facilitate access to and utilization of the associated data stores and resources by the major participants (i.e., patients, doctors, pharmacists, family members etc.) in the health and wellness program.

In accordance with an embodiment of the present invention, the IHMT system has a health knowledgebase that includes medical decision-making intelligent agents, access to clinical research information, and related health databases. Additionally, the IHMT provides resources for registering and coordinating a plurality of patient "health and wellness partners" and providing controlled access to data depositories and resources controlled by the various participants (i.e., primary care physician, endocrinologist, dietician, pharmacist, family members etc.) in accordance with non-reputable agreements, terms of use and the applicable statutes for the parties and jurisdictions involved. This information is remotely accessed using networked terminal devices (i.e., personal computers, network enabled cellular phones, personal digital assistants (PDAs), two way pagers, etc.) by authorized health and wellness participants for the purpose of assisting in health in wellness programs for individuals and groups.

The present invention provides a method for managing diseases and wellness online, the method comprising: receiving patient data over a network from a user regarding a health condition; filtering the patient data according to a first database to produce filtered patient data; performing an analysis of the patient data; and outputting, in response to the received patient data, a medical recommendation of the health condition based on a second database, wherein the medical recommendation includes what the user is suggested to do in regarding to the health condition.

Preferably, the receiving of the patient data comprises: verifying the user by looking up an account associated with the user; requiring the user to set up the account if the account can not be verified; and composing a number of questions based on the first database in conjunction with the account if the account can be verified.

The patient data may include answers from the user to the questions and the patient data may be in the form of diagnostic data received from a diagnostic test device.

The first database may include a common knowledge database about the health condition, the knowledge database being constantly updated with other related servers on the network, and the filtering of the patient data according to the first database comprises discarding some of the patient data that are not so related to the health condition; and requesting correction or verification on other of the patient data when the other of the patient data appears abnormal according to the first database.

The performing of the analysis of the patient data comprises: obtaining statistical features of the patient data through the statistical analysis; determining possible causes related to the health condition out of the patient data in conjunction with the statistical features. The statistical analysis may include a fundamental statistics, a data variability analysis, and a trend forecasting and some of the statistical features by the fundamental statistics may include mean, mode, max, min, ratios and fractions to determine an appropriate sorting algorithm. Also, the variability analysis may determine how significant the patient data is as well as the patient data is distributed and the trend forecasting may include a projection of the patient data, computation of trends with respect to the patient data using one or more mathematical methods. The one or more mathematical methods employed may include one or more of linear and/or non-linear regression techniques, curve-fitting methods and numerical analyses and the performing of the analysis of the patient data comprises, through the medical analysis, evaluating a state of the health condition using a medically related logic, risk stratification, and protocols/algorithms/guidelines that pertain to the health condition. The medically related logic may be a medical modeling logic that simulates a medical decision-making process and is based on general medical decision making principles. Alternatively, the medically related logic is a medical modeling logic that is based on branch/tree logic and hash or hash-like array memory structures.

Furthermore, the second database may be a medical management knowledgebase including static and/or dynamic information from multiple sources pertaining to the health condition and the health condition may include one of a chronic disease and/or a health question.

In a preferred embodiment, the receiving of the patient data over the network comprises: maintaining an account associated with the user; and updating the account with the patient data related to the health condition. The terminal device used to communicate the patient data may include a display screen to display the medical recommendation. Such a terminal device may be selected from a group consisting of a personal computer, a network enabled cellular phones, a portable computing device and a personal digital assistant. The medical recommendation is preferably in a format of a markup language displayable on the terminal device and the composing of the number of questions comprises generating the questions about the user in reference to the health condition and further in reference to the first database.

In an alternative aspect the present invention provides a machine-readable medium embodying instructions for execution by a processor, the instructions, when executed by the processor, causing the processor to produce structured documents, the machine-readable medium comprising: program code for receiving patient data over a network from a user regarding a health condition; program code for filtering the patient data according to a first database to produce filtered patient data; program code for performing an analysis of the patient data; and program code for outputting, in response to the received patient data, a medical recommendation of the health condition based on a second database, wherein the medical recommendation includes what the user is suggested to do in regarding to the health condition.

Preferably, the program code for receiving the patient data comprises: program code for verifying the user by looking up an account associated with the user; program code for requiring the user to set up the account if the account can not be verified; and program code for composing a number of questions based on the first database in conjunction with the account if the account can be verified.

The foregoing and other objects, features and advantages of the invention will become more apparent from the following detailed description of a preferred embodiment, which proceeds with reference to the accompanying drawings.

The present invention will be readily understood by the following detailed description in conjunction with the accompanying drawings that are provided as examples only of embodiments of the invnetion, wherein like reference numerals designate like structural elements, and in which:
**Figure 1** is a block diagram of a communications system which may be used to implement a method and system embodying the invention;
**Figure 2A** illustrates a representative IHMT server device in accordance with a preferred embodiment of the present invention;
**Figure 2B** illustrates a functional block diagram of functions contemplated by an IHMT server or in conjunction with other servers on a data network according to one embodiment of the present invention;
**Figures 3A** through **3J** illustrate representative wireless communication devices (PDAs) displaying graphical user interface screens for interacting with IHMT system in accordance with a preferred embodiment of the present invention;
**Figure 4** is flow diagram of the process associated with receiving patient related data in accordance with a preferred embodiment of the present invention; and
**Figure 5** is a flow diagram of a process associated with the analysis of the received patient data in accordance with a preferred embodiment of the present invention.

The invention pertains to a method, a system, a computer product for managing a multi-domain health and wellness program using remotely located terminal devices, software agents and remotely stored participant information associated with definable access levels. The present invention can be advantageously used to keep users in health and out of crisis. The users may include, but not limited to, patients, medical doctors, caregivers and healthy persons. All desire personalized information and resources to help them keep healthy and active.

In the following detailed description of the present invention, numerous specific details are set forth in order to provide a thorough understanding of the present invention. However, it will become obvious to those skilled in the art that the present invention may be practiced without these specific details. In other instances, well known methods, procedures, components, and circuitry have not been described in detail to avoid unnecessarily obscuring aspects of the present invention. The detailed description of the invention is presented largely in terms of procedures, steps, logic blocks, processing, and other symbolic representations that directly or indirectly resemble the operations of data processing devices coupled to networks. These process descriptions and representations are typically used by those skilled in the art to most effectively convey the substance of their work to others skilled in the art. Reference herein to "one embodiment" or "an embodiment" means that a particular feature, structure, or characteristic described in connection with the embodiment can be included in at least one embodiment of the invention. The appearances of the phrase "in one embodiment" in various places in the specification are not necessarily all referring to the same embodiment, nor are separate or alternative embodiments mutually exclusive of other embodiments. Furthermore, the order of blocks in process flowcharts or diagrams representing one or more embodiments of the invention do not inherently indicate any particular order nor imply any limitations in the invention.

According to one aspect of the present invention, a system, referred to herein as Intelligent Health Management Technology (IHMT) system, provides assistance in regards to the management of chronic diseases in conjunction with multi-domain health and wellness programs. The system collects personal health information and medical record data and analyzes the information, and makes physician-like recommendations based on the available data wherein the recommendations may include computer generated recommendations and/or inputs from participating third parties (i.e., doctors, dieticians, pharmacists etc.). More specifically, the present invention utilizes intelligent agents, network based software application modules having definable access levels, digital credentials, access rules and personalized contact lists to facilitate access to and utilization of the associated data stores and resources by the participants (i.e., patients, doctors, pharmacists, family members etc.) in the health and wellness program.

Dedicated modules associated with given chronic diseases and illnesses are provided in an IHMT system that operates in a computing device (e.g. a server). A given IHMT module acquires and collects patient data related to a health condition (i.e., diabetes, cardiovascular disease, hypertension etc.). The data may be obtained from multiple sources. The data may be entered directly into the IHMT module by a user or loaded by a biomedical device (i.e., a glucose monitor device) or via other third party (a server or a software application). The data may also be provided by other participants (i.e., medical records, doctor comments, dieticians, family members) that may be associated with a given patient's health and wellness program. Data previously stored and/or analyzed may also be retrieved for use by the patient or participants as allowed by agreement and statute. For a given health issue, the data may include information on related health issues as well. For example, medical history information for the cardiovascular module may require the knowledge of whether or not someone has diabetes. The hypertension module may need the information on the state of the user's carbohydrate intake. The IHMT module can ask the user questions such as "How do you feel today?" to obtain subjective data, which can later be quantified. To facilitate the description of the present invention, it is assumed that an IHMT module pertaining to a particular medical condition or illness (i.e. a health condition) is provided in an IHMT system.

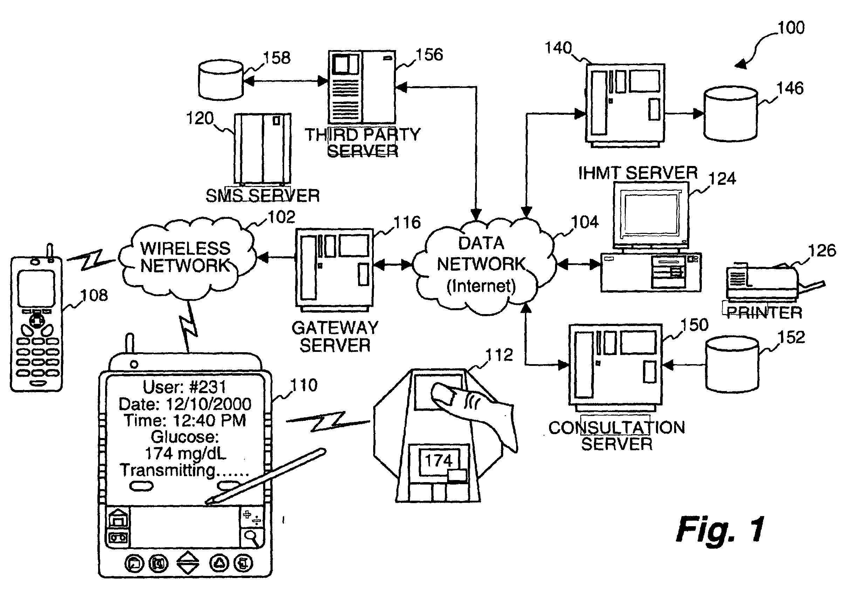

Referring now to the drawings, in which like numerals refer to like parts throughout the several views. **Figure 1** shows an exemplary system configuration in which the present invention may be implemented in accordance with a preferred embodiment. Multi-domain health and wellness communications system **100** generally includes a plurality of communications networks such as Intranet/Internet **104** and wireless network **102.** These communications networks support communications between a plurality of diverse terminal devices as illustrated by network enabled cellular telephone **108,** wireless PDA **110** and personal computer **124** having differing communication protocols and operational parameters. A portable diagnostic test system **112** is used to measure one or more biomedical parameters and transfer them to one of the terminal devices. In one possible configuration, a portable diagnostic test system **112** can be configured to communicate with one of the servers (e.g. IHMT server device **140**). For example, Glucometer® Dex® Diabetes Care system by Bayer™, as one exemplary diagnostic system, can be used measures blood glucose levels, and provide facilities for uploading the results to a selected terminal device. Similar systems are available from other providers and for other chronic illnesses. A gateway server **116** facilitates intra-network communications. Server devices such as IHMT server **140,** consultation server **150,** and third party server **156** may be also coupled to network **104** and perform other service functions related to the management and utilization of the information retrieved from diagnostic test system **112** and data retrieved from other sources as will be further described below.

IHMT server device **140,** such as a network connected SUN workstation or NT server, includes storage means **146** for storing patient data for a plurality of patients and/or participating subscribers or users and hosting one or more medical knowledge bases in addition to medical intelligent software agents configured to provide recommendations based on inputs from a user. In one aspect, IHMT server **140** manages subscriber information and coordinates interactions between other participant domains such as participants in the subscriber's health and wellness program and interested third parties such as pharmaceutical companies. IHMT server **140** will be discussed in further detail below.

Consultation server device **150,** such as a network connected SUN workstation or NT server, includes storage means **152** for storing, patient related data not under the control of the patient (i.e., medical records, laboratory data, prescription data, etc...) and participant information (i.e., physician appointment schedules). Medical records by their very nature generally require a higher level of security (i.e., server physical security and firewalls) than most archived information. Information may be exchanged between the two domains in accordance with the prevailing legal statues and agreed upon terms of use. For example, the patient's endocrinologist might make a portion of the patient's medical record available to IHMT server **140** and have access to the medical data stored of the IHMT server associated with the subject patient. Additionally, the patient's endocrinologist could make his/her appointment schedule available through the IHMT and could set test result trigger points where office visits are proactively suggested (i.e., via email or telephone) when a predefined limit is reached (i.e., prolonged elevation of blood glucose levels).

Third party server device **156,** such as a network connected SUN workstation or NT server, includes storage means **158** for storing patient data, and commercial service offerings and information relating to the patients condition (i.e., drug recall notices) which could be downloaded and printed using personal computer **124** and printer **126.** The information provided to and utilized by third party server **154** may be sanitized to remove patient identification information.

The descriptions of IHMT server **140,** consultation server device **150** and third party server device **154** provided above, are provided for purposes of illustration and not limitation. It would be understood by those skilled in the art that it is not necessary to have to implement each of the servers, parts or devices as illustrated in **Figure 1** in order to practice the present invention and the system components may differ from those described or illustrated above but still provide functions contemplated by the present invention.

According to one embodiment, IHMT server **140** can be configured to receive inputs from a coordinator planning a conference consultation for a user. Software agents resident in IHMT server **140** generate invitations for the requested participants with respect to the inputs received from the coordinator. The inputs may include associated participant information (i.e., attributes, schedules and user defined information access limitations). The generated invitations may be forwarded to selected participants by, perhaps, a voice channel (wireless or land-based) and via SMS server **132** and an associated narrowband channel or via e-mail. Consider a scenario in which a patent participant in a health and wellness program for a chronic illness, such as diabetes, requiring long-term preventative maintenance. The patient may have multiple caregivers (i.e., a primary care physician, an endocrinologist, a podiatrist, a dietician and a pharmacist). All the participants in this health and wellness program have information that may be of value to other participants. The problem arises because the necessary information resides in distinct domains. According to the embodiment, the server coordinates interactions among the various domains. All the participants in this process may be associated with a set of digital credentials and associated access rights and "terms of use" which are used to validate and control interactions with protected resources.

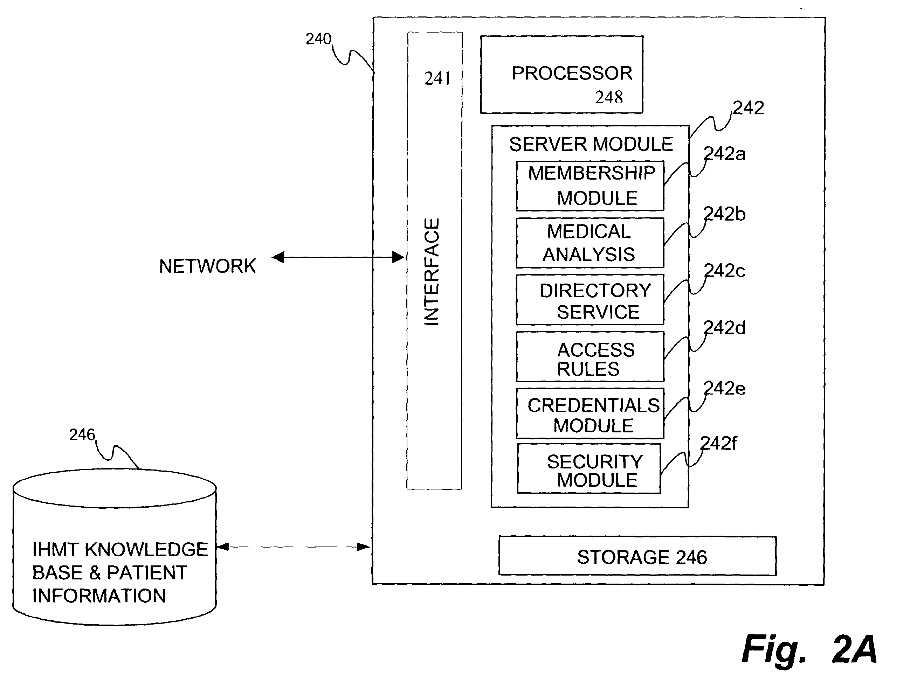

Referring to **Figure 2A,** there is shown a functional block diagram of IHMT server **240** that may correspond to IHMT server **140** of **Figure 1.** A network interface **241** facilitates a data flow between a data network (i.e., data network **104** of **Figure 1**) and IHMT server **240** and typically executes a special set of rules (a protocol) for the end points in a link to send data back and forth. One of the common protocols is TCP/IP (Transmission Control Protocol/Internet Protocol) commonly used in the Internet. The network interface manages the assembling of a message or file into smaller packets that are transmitted over the associated data network and reassembles received packets into the original message or file.

In addition, IHMT server **240** comprises a processor (or multiprocessor) **243,** an IHMT server module **242** and a storage space **246.** In practice, any computing device having reasonable computing resources (i.e., processing power and memory capacity) may be utilized as an IHMTserver. Storage space **246** may be resident within IHMT server **240** or in a separate accessible server device (not shown). Part of the storage space **246** is allocated to retain patient related information uploaded from one or more client devices and accessible upon request for requestors having the appropriate credentials or access rights. It should be noted that the storage space **246** may be a single storage device or a cluster of storage devices located locally and/or remotely (i.e. connected through a network). In one embodiment, storage space **246** retains patient data respectively associated with a number of particular patients or users.

According to one embodiment of the present invention, IHMT server module **242** is a compiled and linked version of a computer language implementing the present embodiment and loaded in a memory. When executed by processor **243** in IHMT server **240,** server module **242** performs a number of functions or operations contemplated by the present invention.

Server module **242** comprises a membership module **242a,** medical analysis engine/module **242b,** directory service module **242c,** access rules module **242d,** credentials module **242e** and security module **242f.** Membership module **242a** provides account initialization, management and service functions for a plurality of user accounts, each preferably for one patient or user. With an established account in membership module **242a,** a user may log on IHMT server **240** from anywhere at anytime from any device capable of data communication with IHMT server **240.** In one embodiment, membership module **242a** is an interface selectively accessible by users or an administrator. Typically, a user is permitted to retrieve those data records associated with his/her own diagnostic measuring and/or recording activities while an administrator is permitted to retrieve or access certain portion of any one's data in storage space **246.**

Medical analysis engine/module **242b** provides physician-like recommendations based on the available data where the recommendations may include computer-generated recommendations and inputs from one or more participating third parties (i.e., doctors, dieticians, pharmacists etc.). The methodology for this analysis will be described in further detail below.

Directory service module **242c** facilitates secure access to sensitive information held in multiple domains. In one embodiment, directory services enable access to hosted repositories of certificates, privilege data and certificate revocation lists (CRLSs). An X.500 Directory Model is employed and is a distributed collection of independent systems which cooperate to provide a logical database of information. In another embodiment, security and privacy protocols promulgated by the Health Information Portability and Accountability Act (HIPAA) may be used to coordinate or access multiple databases. It is understood to those skilled in the art that other commonly acceptable protocols may be used. It should be pointed out that it is not a requirement for the present invention to operate on specified standards. Adherence to standards makes the distributed model more efficient. It is possible for one organization to keep information about other organizations, and it is possible for an organization to operate independently from the global model as a stand-alone system.
Registries containing information that is related to an individual is freely transferred and unregulated in the US, unless the provider of the data is an agency or an holder of sensitive information as defined by federal legislation and further may differ for each state. Medical records fall into the class of sensitive information and therefore a flexible means for providing access to this information is preferred.

Access rules module **242d** contains rules relating to "terms of use" for access to sensitive information. If a party required access to data not under their direct control then that party must agree to the terms of use for the requested data or resources. Agreement is by non-reputable means such as an electronically signed agreement.

The last two modules relate to system security. Credential module **242e** coordinates activities relating to the distribution and validation of electronic credentials (e.g. with the assistance of a Certification Authority (CA) and a Registration Authority (RA)) in accordance with procedures that are well known by those of ordinary skill in the art. Security module **242f** is associated with IHMT server level security. It is understood to those skilled that the exemplary functional blocks in server **240** would make the present invention more efficient, however, not each of the blocks must be implemented in order to practice the invention.

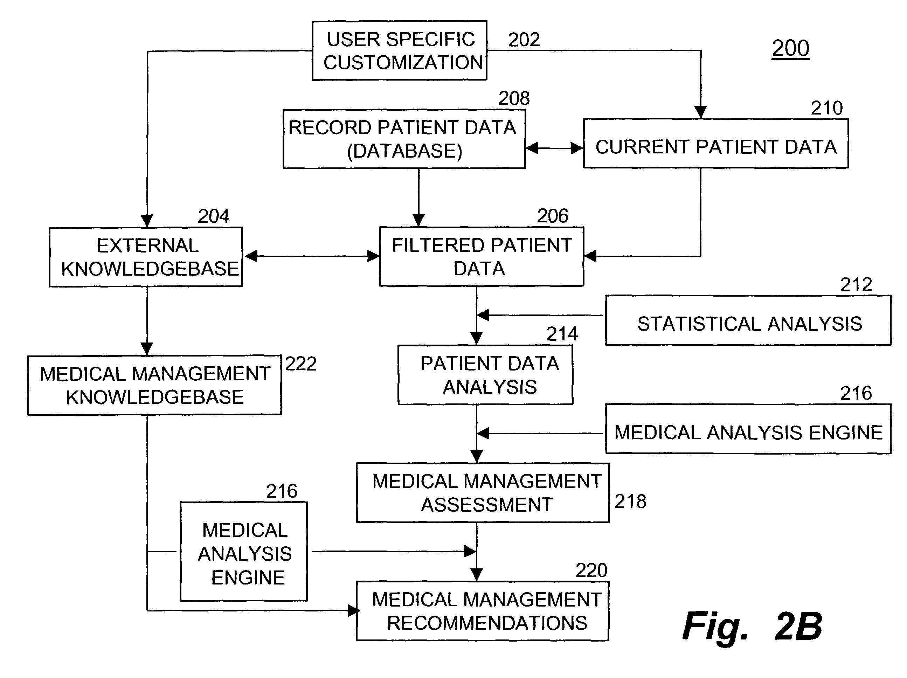

Referring now to **Figure 2B,** there is shown a functional block diagram **200** of functions contemplated by an IHMT server or in conjunction with other servers on a data network according to one embodiment of the present invention. Each of the modules or blocks may be implemented in software, hardware or a combination of both and preferably operate in a computing device (i.e. a server) capable of data communications over a data network. The details of the computing device are well known and not to be described herein to avoid obscuring the aspects of the present invention.

Before describing functional block diagram **200,** it deems necessary to provide definitions to some of the terms used herein to facilitate the description of the invention:

Patient data: any data related to a patient or person utilizing the IHMT. This may include, but not be limited to, medical/health information and demographic information in both objective and subjective forms. For example, in a diabetes IMHT module, patient data may include objective data such as the blood glucose level of the user or the viral load of an HIV patient, and etc. Subjective data such as "how the user is feeling at the moment" may be acquired for use by the IHMT module.

IHMT module: IHMT may be configured or implemented in a modular fashion, each module is focused on a specific disease, medical condition, or wellness issue, referring to a health condition herein. Examples of the modules may include IHMT applications for diabetes, asthma, hypertension, pregnancy, HIV/AIDS, parenting issues, dieting, nutrition, fitness, exercise, smoking cessation, weight loss, travel health, allergies, arthritis, heart disease, medications, etc. IHMT modules can also be built as "decision support applications" for healthcare providers, physicians, hospitals, insurance, etc. IHMT modules may communicate with each other and interchange data with each other. In a preferred embodiment, each module is a software application or module and interoperable with each other.

Knowledgebase: an information database comprising static and dynamic information from multiple sources, databases, online or offline resources. For example, the Medical Management Knowledgebase includes articles regarding health issues, databases of online resources, databases of educational resources, database of interventions, database of community sources, database of healthcare resources, and so on. The databases may be relational or object-oriented databases.

User Customization: This pertains to the fact that the IHMT modules are personalized to individual users, based on such characteristics as location, age, sex, race, medical conditions, and so on.

Medical: The term "medical" is used to describe any health-related issue, which includes diseases, medical conditions, and wellness issues.

Medical Management: This pertains to the tending of a medical or health issue. This may include physicians managing a patient's disease or wellness or a person taking care of their own health-related issue, or a person taking care of someone else's health-related issue.

Medical Aspect: This relates to a specific issue that is related to the management of the health-related issue. For example, the average peak flow level over the past 14 days is an aspect of asthma. Other examples include: the symptoms a person is feeling; the current pollen count in a certain location; the blood glucose level of a patient at a certain time; the trend in the blood pressure; the cyclical variation of user data; the ethnicity of a patient; the background medical history for a patient; the date of birth of the user's child; and etc.

Recorded Patient Data: This generally takes the form of a database (object-oriented and/or relational). This includes all pertinent information related to the user's of IHMT modules, including health and non-health information.

Terminal devices, also referred to as networked terminal devices herein, include but are not limited to personal computers, laptop computers, computer terminals, personal digital assistants, palm-sized computing devices, and networked wireless communications devices such as micro-browser enabled cellular telephones. Such devices typically have a user interface including a display, an input interface (i.e., a keypad) and a pointing device (e.g., a mouse, a trackball, a joystick, a navigation key-set or a touch-pad).

An input mechanism **202** is provided to a user to input or upload various data and customize an account thereof. The received data is typically stored in memory as current patient data **210.** In accordance with one embodiment, a participant is registered in a health and wellness program and needs to answer a number of questions displayed on a screen of a terminal device. The questions are transported over a data network as one or more web pages (e.g. HTML) from an IMHT server and may include generic and/or specific personal questions. The answers provided by participant are transported back over the network to the IMHT server to customize and update the account associated with the participant. In accordance with another embodiment, the mechanism **202** permits a user to define access levels for available data resources from or through the IMHT server. For example, a no-fee structure may access a first level of data (e.g. preventative wellness program) and a fee structure may access a second level of data (e.g. recommendations from specialists). In one aspect, the user defined access levels may act as selective information filters for the information utilized to setup and/or implement crossing consultation/ conferences on a particular illness or subject. Still in accordance with another embodiment, a user may upload diagnostic test data to the IMHT server through mechanism **202** to support the answers provided to the questions being asked. Depending on an exact application, the inputs from a user may vary. Unless specifically stated, the inputs from the user or participant are collectively herein referred to as patient data.

Patient data received from mechanism **202** are to go through a knowledgebase **204** to generate filtered patient data **206** that is typically to update recorded patient data **208** in the account. In one embodiment, knowledgebase **204** is periodically updated from other resources on the network and used to filter out or discard some of the inputs from the user that may not be related to a particular illness or a subject. Examples of the other resources may include various medical resources, latest discovery and recommended diagnose of a particular health condition as such knowledgebase **204** can generate filtered patient data **206** that can be relied upon by subsequent medical analysis. In another embodiment, knowledgebase **204** is referred to customize the account. For example, a user has suffered from asthma for years. After the user is registered with the IMHT server, an account is established therefor. Based on the initial data provided, the account may include a set of customized questions related to the disease in reference to knowledgebase **204** so that the questions are much more related to the illness the user is suffering.

Filtered patient data **206** may be verified or entered from previously recorded patient data **208.** Filtered data **206** is then analyzed or reviewed by, perhaps, a statistical analysis **212** to ensure that the data is true and sensible. Errors can be noted if an error or invalid data is observed, proper means may be used to reacquire the information. Different statistical analysis may be applied to depending on an exact subject (e.g. an illness). In one embodiment, statistical analysis **212** is implemented based on a survey among a group of similar people with respect to the same or similar subject in the filtered data. Other possible statistic analysis, such as fundamental statistics, data variability analysis, and trend forecasting may be utilized. Fundamental statistics may include, but not be limited to, such analysis as mean, mode, max, min, ratios, fractions, sorting algorithms, application of mathematical formulas, and etc. Variability analysis may include; but not be limited to, analysis such as tests for significance of data, distribution of data, and etc. Trend forecasting may include, but not be limited to, all analysis related to projection of data, computation of trends, linear and non-linear regression techniques, curve-fitting methods, numerical analyses, etc. When filtered data is analyzed and processed in the IHMT, the process is referred to as patient data analysis **214.**

According to one embodiment, a medical analysis engine **216** generates a Medical Management Assessment **218.** Medical analysis engine **216** includes modules or components that evaluate the state of the medical condition or health issue, medically related logic, risk stratification, and protocols/algorithms/guidelines that pertain to the medical issue at hand. In the embodiment, Medical Modeling Logic is used in medical analysis engine **216** to aid in making appropriate or customized decisions. The medical modeling logic simulates medical decision-making process and is based on general medical decision making principles, common sense principles, and specific logic for the given IHMT module. These principles may be derived from various sources such as standard medical practice guidelines, clinical research, mathematical relationships, biologic relationships, and consensus recommendations. By using correlation analysis, the medical modeling logic relates significant trends, data points, and other factors to enable causal analysis.

In one preferred embodiment, the medical modeling logic is in the form of branch/tree logic and/or the form of hash or hash-like array memory structures for more efficient or accurate decision-making. Typically, branch/tree logic is used for simple decisions; whereas, the hash or hash-like array memory structures (H-logic) are used in complex decisions requiring N-axis of information, where N can be any non-negative integer. The H-logic requires preformed endpoints that are stored or preloaded into the memory architecture of the computer. Some endpoints may be dynamically generated earlier in the decision-making scheme and may include recursive elements. H-logic may arrive at its decision using parallel and/or non-parallel processing of data. The data is used to localize up to N endpoints for N-axis of information. In some cases, less than N endpoints for N-axis of information are required. H-logic is more efficient and faster and producing decisions than branch/tree logic related algorithms.

In operation, medical analysis engine **216** evaluates the current and projected state of the given health-related aspect or issue with respect to the received patient data (filtered), which sometimes is referred to as "Medical State Evaluation". One example is that medical analysis engine **216** may decide that a certain blood glucose level is "high" or "low" or will become "high" or "low" with reference to the received patient data. One component in medical analysis engine **216** is referred to as "Risk Stratification component" that uses the medical modeling logic and medical care protocols to relate to the Medical State Evaluation by evaluating the state and determining the importance of the state and quantifying the state. The result of the Risk Stratification component may be used by the Medical State Evaluation for the current analysis or later analysis. Likewise, the result of the Risk Stratification component may be used to modify or select the appropriate Medical Care Protocols to be used. In certain instances portions of the Medical Analysis Engine like the Medical Care Protocols may be customizable by users, health care providers, or others. For a given health issue or medical condition, there may be many aspects each of which is analyzed by the Medical Management Assessment **218.** In one embodiment, medical management assessment **218** first identifies pertinent aspects to the medical or health issue and then proceeds to analyze them using medical analysis engine **216.** The result is an assessment of each medical aspect for the health-related issue and its related issues. In the embodiment, the Medical Management Assessment **218** evaluates the state of each subset of data related to a given health condition using Medical State Evaluation component of the Medical Analysis Engine. For each subset of data, the Medical Analysis Engine **216** then performs appropriate analysis techniques to identify the trend of the target data. Correlations between trends, subsets of data, and other factors are then identified based on Medical Modeling Logic segment of the Medical Analysis Engine. Once causal relationships and correlations are established, these findings are identified within the Medical Care Protocols as governed by the Medical Modeling Logic to provide the appropriate output that governs the Medical Management Assessment as well as the Medical Management Recommendations.

An example of one embodiment of the Medical Management Assessment within the IHMT Diabetes module is now described. Given a glucose value for a patient, the Medical Management Assessment uses the Medical Analysis Engine and initially identifies whether the value is "high" or "low." Subsequently, it identifies trends and patterns of the glucose variation in relation to time, dietary patterns, and other factors. Once these analyses have been performed, the Medical Management Assessment can identify causes for these variations and then provide subsequent appropriate clinical and lifestyle recommendations, solutions, and positive/negative feedback as derived from the knowledgebases. The IHMT Diabetes module can now generate a complex assessment regarding the patients blood sugar control such as: "Your blood sugars appear to be rising over time and may reach a critical value in three days. Your overall control of your blood sugars is poor. In other words, you're diabetes is out of control. This may be due to the recent dietary changes and lack of compliance with the medications." The medical management assessment **218** then proceeds to analyze all aspects of the health issue (in this case diabetes) including projected/forecasted states and the produce additional assessments of the patient's management of his/her disease or health issue.

Results of the medical management assessment **218** are then combined with the appropriate portions of Medical Management Knowledgebase **222,** perhaps via medical analysis engine **216.** One of the features in the present invention is that the medical management recommendations are dynamically created for the particular medical condition after the above analysis and consultations with the related knowledgebase. Depending on an exact implementation, the output (i.e. the medical management recommendations) may be in the form of a written or graphical report and/or dynamic function or action, presentable to a browser or a display engine. In one embodiment, one aspect of the medical management recommendations is configured to initiate a contact of the user's physician or related specialist if needed so that necessary care can be provided to the user in a timely manner. Generally, the output is stored with the user's account for future reference and can be accessed by authorized personnel upon request. Medical recommendations include clinical and lifestyle interventions, care plan adjustments, follow-up guidelines with health care providers, positive and negative reinforcement, learning suggestions, forecasts and warnings regarding the patients health condition. Medical recommendations are derived from the knowledgebases which are described as follows.

Generally, medical management knowledgebase **222** may include, but not be limited to, medical physician/provider databases, online learning databases and classes, medical communities, medical intervention databases, related Internet databases, medical resource databases, and medical education databases. Essentially, the medical management knowledgebase contains related knowledge to the given disease or health issue for each IHMT. It is evident to those skilled in the art that medical management knowledgebase may contain static and/or dynamic resources.

According to one embodiment, the medical management knowledgebase **222** includes a component referred to as medical intervention database that includes short-term intervention, long-term intervention, and physician/provider follow-up recommendation components. Interventions can be customized by the user or others such as the user's physician, user's relative, or the user's environment. Interventions can be in the form of "Tip of the Day" and/or report format. The Interventions may target specific individuals; in other words, they may be categorized based on the user's level of knowledge such as novice, intermediate, and advanced. Interventions are also prioritized in terms of importance and/or possible impact on the user's health state.

A short-term intervention usually applies to the current time, namely, this intervention addresses what the user should do now or in the near future. For example, if a patient has high blood sugars today, a short-term intervention may be "Inject 2 units of Regular Insulin subcutaneously." Long-term interventions address goals that the user should strive for; the intervention may take time to achieve or implement. For example, a long-term intervention for diabetes may be something like: "Your diabetes may improve if you lose another 10 lbs."

Physician follow-up recommendation relates to relationship between the user and their physician or healthcare provider and any information the user may need to communicate to their physician. One example is: "We recommend that you speak to your physician about adding a second generation oral hypoglycemic medication to your evening regimen. Please follow-up with your physician to optimize your treatment medication in 1 to 2 weeks." Another example is: "Your asthma is critically severe. Please dial 911 or go to the nearest emergency healthcare provider now."

The Interventions may also include dynamic functions as well. For example, in cases of emergency, the intervention may actually link the patient to emergency care provider by contacting 911 or faxing/emailing/paging critical data to a healthcare provider designated by the user's environment. Another example may be that the information is transmitted to the user's pager as a reminder for certain health issues such as "Don't forget to measure your blood sugar" or "Reminder: take your diabetes medication now."

The output may be also produced in conjunction with other databases incorporated in medical management knowledgebase **222** or external knowledgebase **204.** For example, one of the databases may be related to weather conditions around the area that the user lives. Given the appropriate conditions and assessments, The output may include the medical recommendations based on the weather information: "Due to your asthma triggers, we note that you should try to avoid the outdoors in the next few days because of the increasing pollen count."

Optionally, another related database may be incorporated to produce: "For more information about pollen and your asthma, visit this website (i.e. a hyperlink)". In addition, the related database may conduct a dynamic search for the user on the Internet using a parallel search algorithm with medical thesaurus on multiple online resources simultaneously and then output the result. Additional output may include: "Because of your pollen trigger, you should enroll in the online learning class called Asthma 202: All about Pollen." According to one embodiment, knowledgebase **222** and/or external knowledgebase **204** may be configured to include data acquired from dynamic searches on community resources. The community resources may include, not be limited to, related articles, related topics in chat rooms or discussion rooms on the Internet, and dynamic search results identifying matches with the user and other users who have similar medical and/or non-medical interests. For example, a recommendation output may include: "Other users in your community such as Bob153 and Sally555 also have diabetes and are interested in discussing these issues."

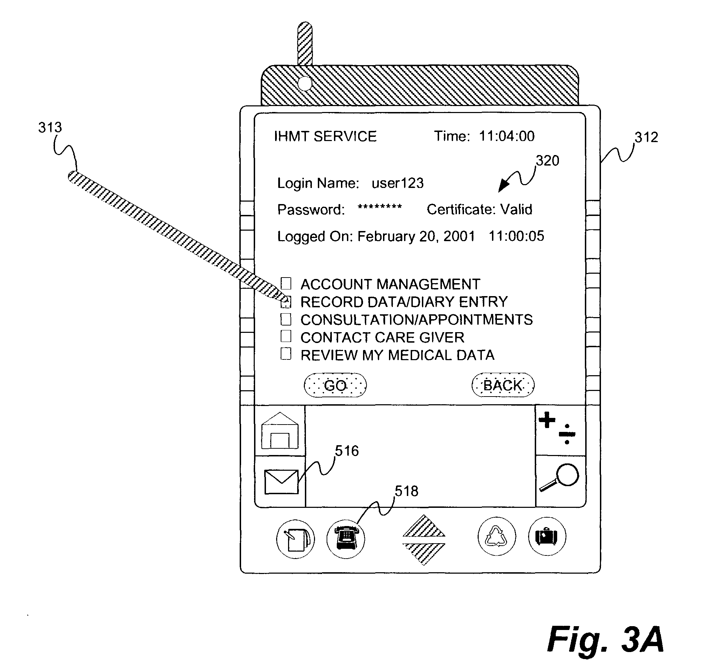

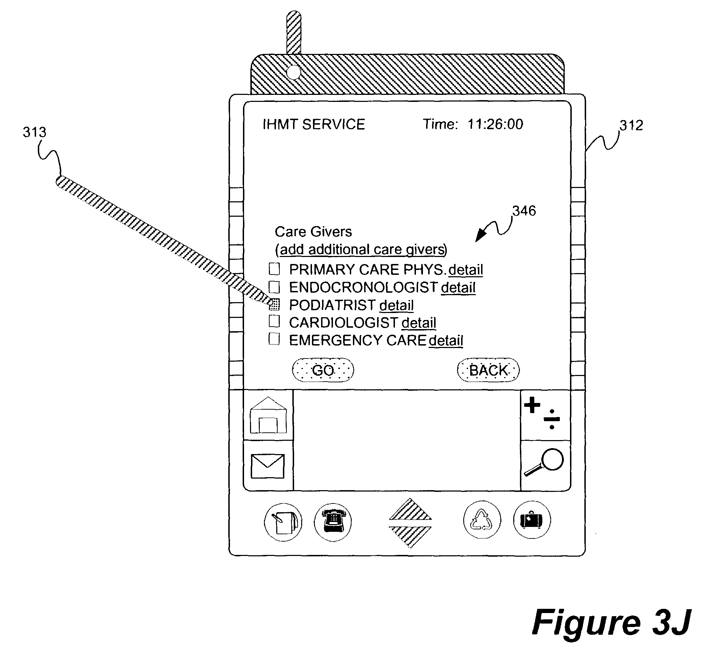

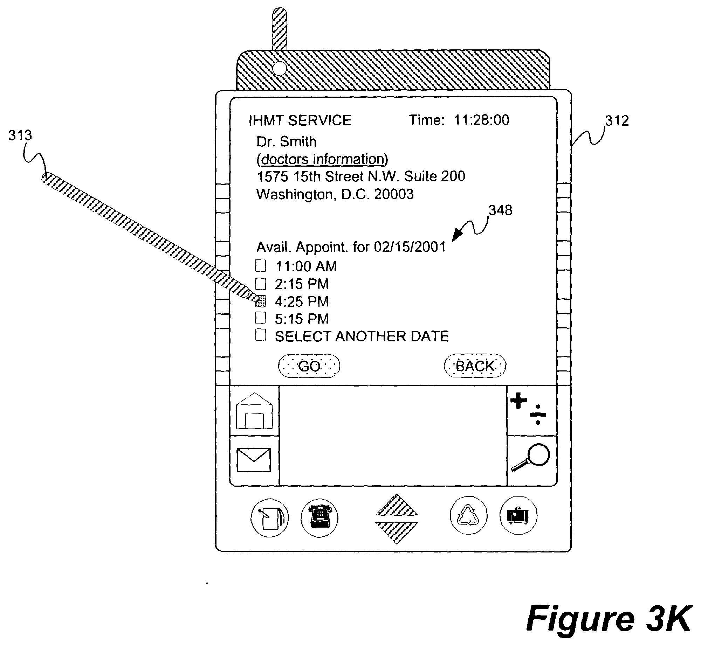

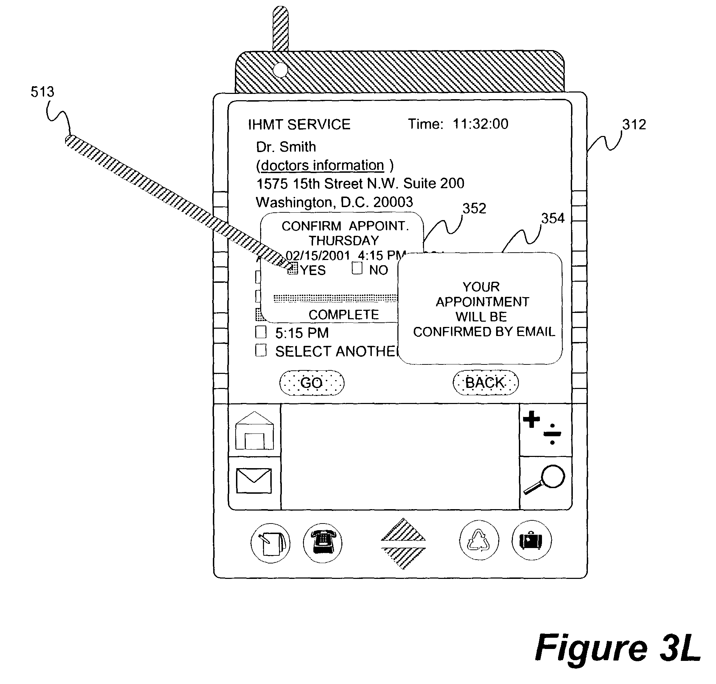

Referring now to **Figures 3A** and **3L,** there are illustrated respectively exemplary user interface screens associated with a wireless enabled PDA **312** which may be used by a patient associated with a health and wellness program to upload their diagnostic test results and to coordinate interactions with other participants and resources and finally receive a recommendation from the IHMT, which may be also referred to as a "virtual doctor" or "virtual caregiver". It is important to note at this point that the terminal device may also be a personal computer or some other wireless communication device such as a network enabled cell phone (i.e., WAP or I-mode).

**Figure 3A** shows a user interface screen **320** that enables a patient participant to access a graphical user interface (GUI) associated with a particular function or subject. As described above, each user may define a different access level the IHMT. These user defined access level acts as selective information filters for the information utilized to setup a corresponding account. It is now assumed that User interface screen **320** pertains to one particular access level, different level may present different user interface screen and different information. I

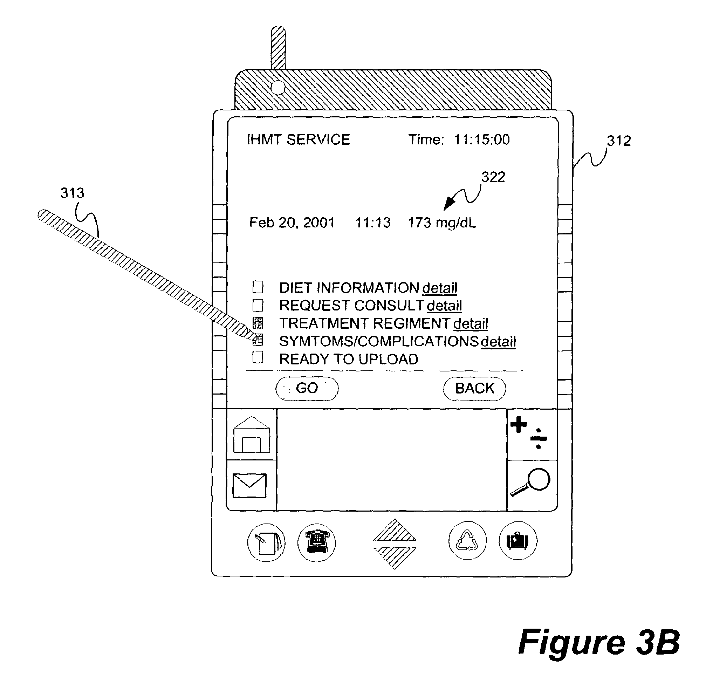

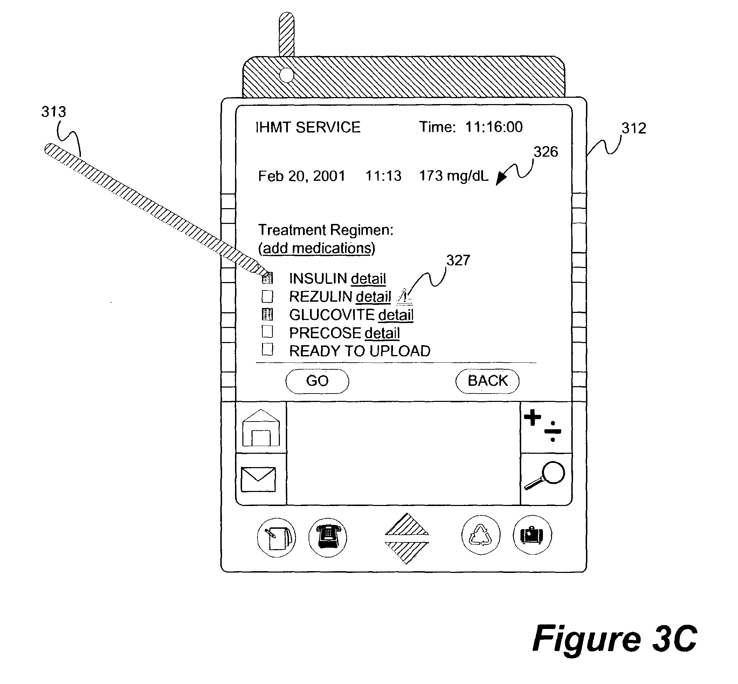

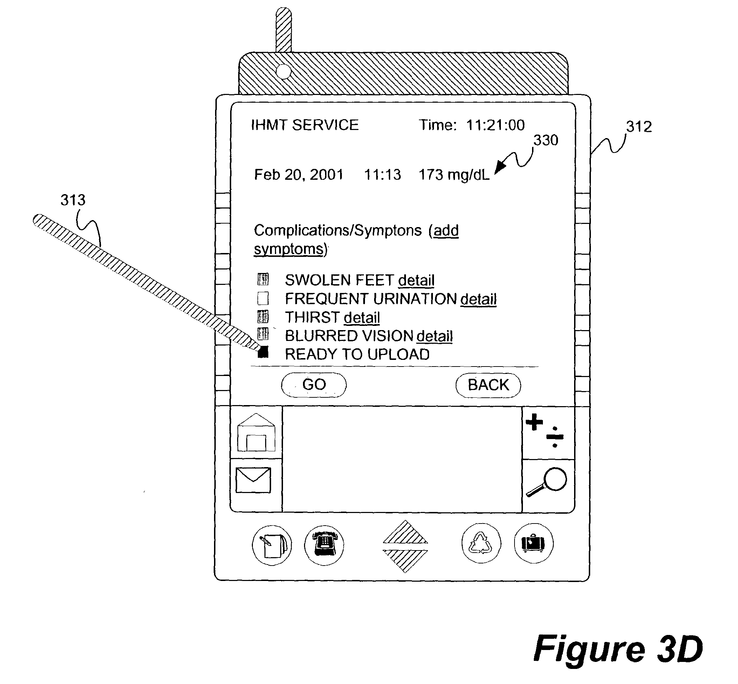

User interface screen **320** includes a series of check boxes, links and softkeys that enable navigation and option selection. The GUI in **Figure 3A** may be used to manage an individual account, enter diagnostic test results, obtain consultations (i.e., from intelligent agents or health care providers) and schedule appointments. In the example illustrated in **Figure 3A,** the checkbox for <RECORD DATA/DIARY ENTRY> has been selected. Activation of the <GO> softkey will cause the GUI illustrated in **Figure 3B** to appear. Referring now to **Figure 3B,** user interface screen **320** enables a patient participant to interact with a GUI associated with adding supplemental information to the diagnostic test results and requesting supplemental information or consultations from health and wellness care givers (i.e., primary care physician, endocrinologist, podiatrist, dieticians etc.) or from automated software agents. User interface screen **322** includes a series of check boxes, links and soft keys that enable navigation and option selection. In the example illustrated in **Figure 3B** the checkboxes are associated with <TREATMENT REGIMEN> and <SYMTOMS/COMPLICATIONS> have been selected. Activation of the <GO> softkey will allow the patient to sequentially interact with the GUIs illustrated in **Figures 3C** and **3D.**

The GUIs illustrated in **Figures 3C** and **3D** enable patient participants to enter information related to their current medications and any relevant symptoms they may have noticed. Additionally, links to important information relating to their medication or diet may be added to the appropriate screens. For example, the link indicated by symbol **327** may provide information about the recall of REZULIN by the FDA.

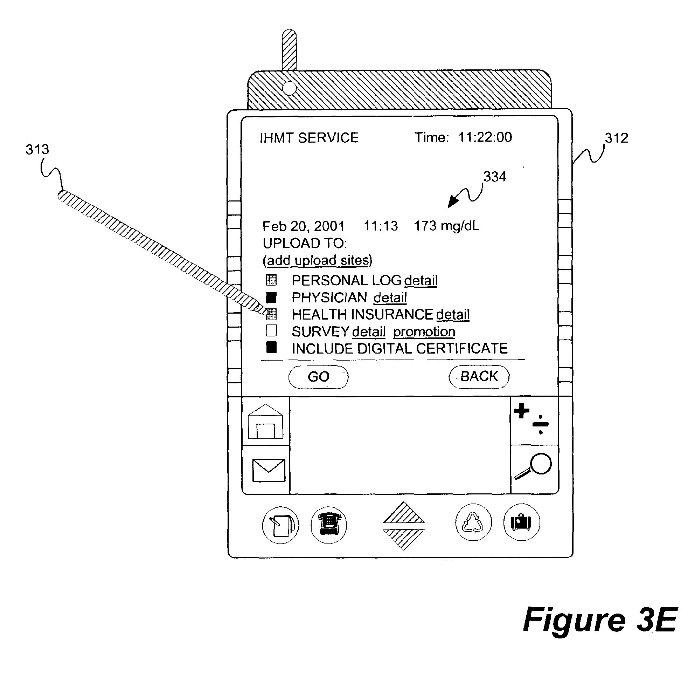

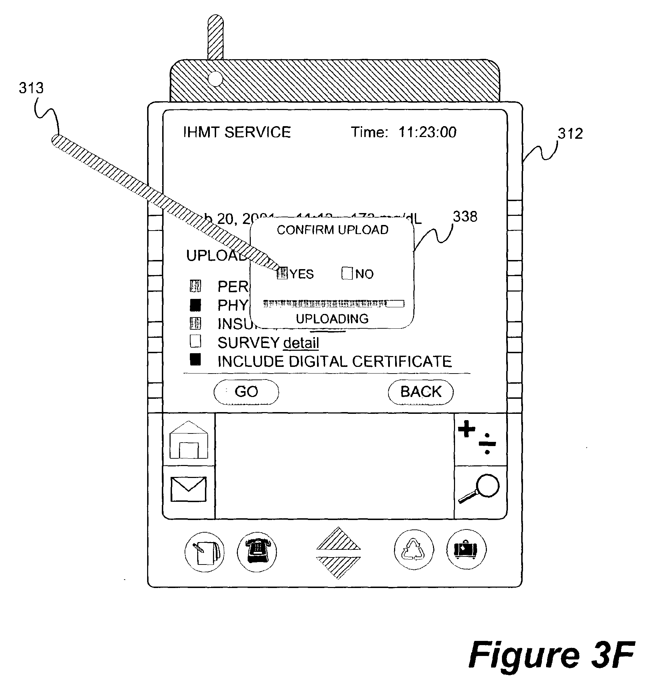

The GUIs illustrated in **Figures 3E** and **3F** enable patient participants to upload and filter information that is to be made accessible to the various participants in the patients health and wellness program. Additionally, any results and the associated access permissions may be signed using the patient's credentials (i.e., a digital certificate) thereby creating a legally recognized audit trail.

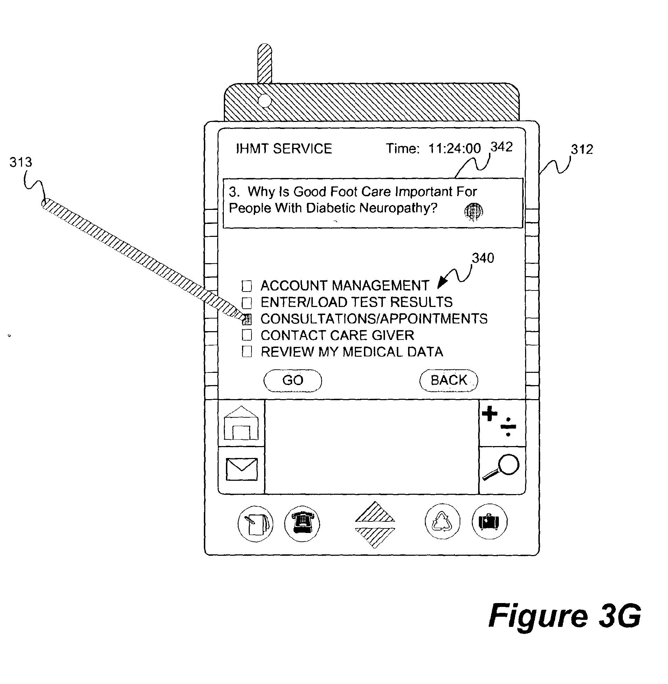

The GUI illustrated in **Figures 3G** enable patient participants to access customized information particularly relevant to their particular disease and symptoms. As illustrated by information link **342,** the particular article selected has particular relevance to the patient participants uploaded results and symptoms.

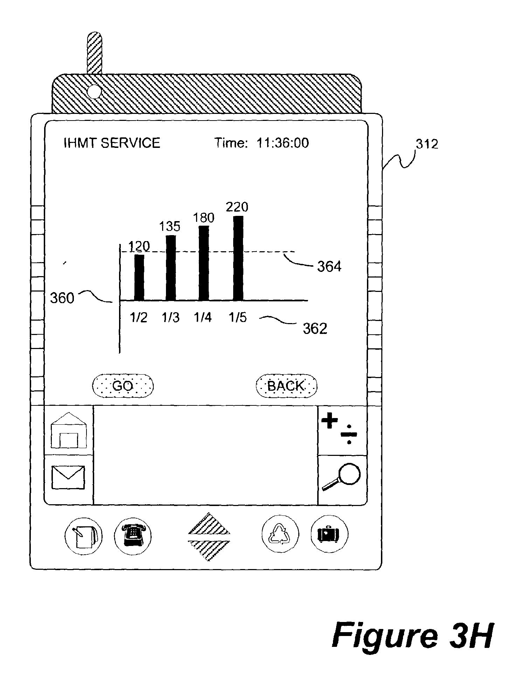

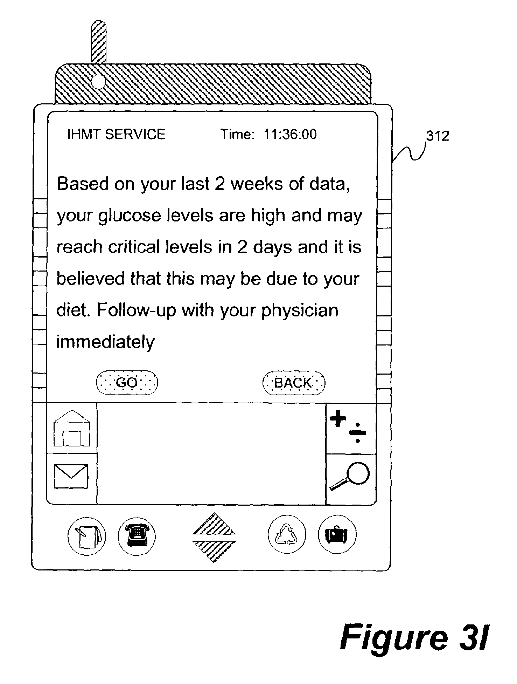

The GUIs illustrated in **Figures 3H** and **3I** show respectively exemplary results provided by the IHMT server. After the patient data is entered and transported to the IHMT server, the data is medically analyzed as described above. A graphic representation **360** is provided to the user for easy understanding of what may have happened to, for example, his/her recent glucose levels with respect to a normal level **364** and corresponding to dates **362.** The example makes it evident that other possible representations may be provided, such as one or more tables, graphs with the abnormal data highlighted in various fashions. When there are some data that appear abnormal with respect to the user's history and/or sample data collected from a group of similarities, the IHMT server is configured to provide a recommendation as shown in **Figure 31** as such the user becomes aware of what he/she shall do to avoid worsening his/her health condition being checked.

The GUIs illustrated in **Figures 3J** through **3L** enable patient participants to access the resources held by the health care providers associated with their health and wellness program. Of particular interest is the ability of this system to access the appointment schedules associated with healthcare providers. This feature provides an efficient and convenient means to set up an appointment. Additionally, priority access to appointments may be provided to those patients having diagnostic test results that are indicative of pending problems.

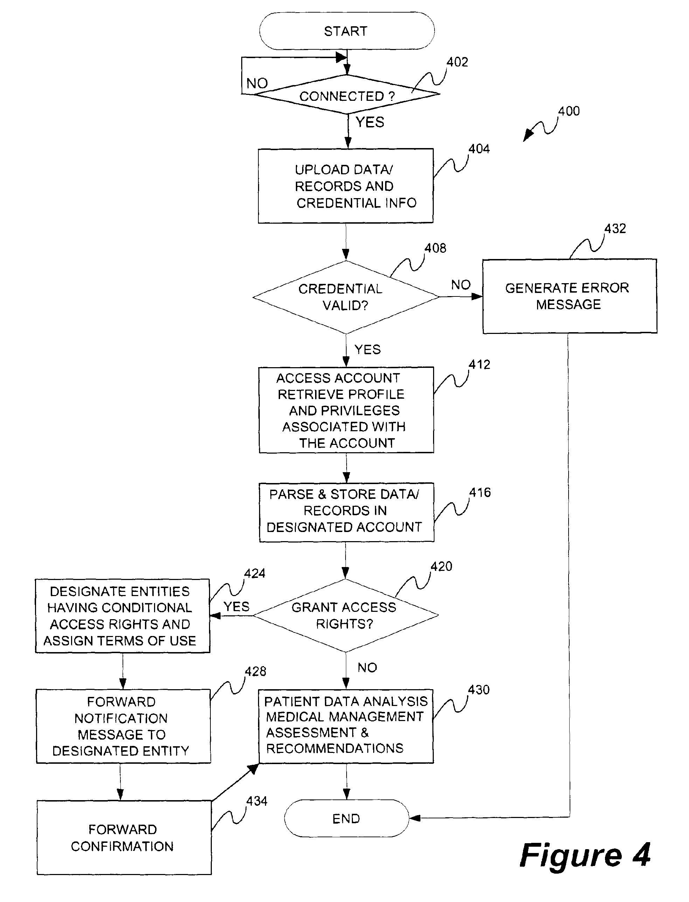

**Figure 4** is flow diagram of a process **400** associated with an IHMT server communicating with a terminal device that may be used by a patient or user. Process **400** may be implemented as a method, a process, a computer product and apparatus in accordance with a preferred embodiment of the present invention and shall be understood in conjunction with the preceding figures. At **402,** the terminal device is first caused to establish a data link with the IHMT server. This may be accomplished by launching a browser (e.g. Microsoft Internet Browser) in the terminal device. After the user enters an address identifier (e.g. an IP address) identifying the IHMT server, the browser sends out a request (e.g. an IP request including the IP address). Only is a data link established with the IHMT server, process **400** may proceed.

At **404,** as part of patient data inputting process, diagnostic test results and/or medical records are to be received from the user. To ensure that the received data will be incorporated into the correct account, the IHMT server is configured to ensure that the user is what he/she says. In one embodiment, a piece of credential information (i.e. username and password) is used. In another embodiment, the credential information is an e-signature (i.e., a digital certificate such as an X.509 Certificate or similar non-reputable electronic record) that is used in association with a request to add the information to a designated patient account. At **408** a determination is made as to whether the received credential information is valid. If the credential information is found invalid, an error message may be generated at **432** and the message may be sent to the user to ask for new credential information or the process **400** is concluded. If the credential information is valid, the designated user account is accessed, the associated user profile information and any associated privileges in the account are retrieved.

At **416** the received data is processed in accordance with the retrieved profile information and privileges. For example, the patient may have it in his/her profile that his/her diagnostic test results should be sent to his/her primary care physician. At **420** a determination is made as to whether the patient participant wishes to grant additional access rights for other program participants (i.e., pharmacist or dietician). If the patient participant desires to grant access rights to additional entities then the required information is gathered and processed at **424.** Notification for the newly designated entities are prepared and forwarded at **428** and upon completion of the upload of the subject information, a success message is forwarded to the appropriate parties at **434** and the process goes to **430.** If at **420** the patient does not desire to designate additional entities having access privileges, the process proceeds to **430** as well.

At **430,** the received patient data is analyzed, as described above, through the statistical analysis. The data is then further applied to the medical engine that provides a medical assessment on the received data concerning a subject. Together with medical management knowledgebase, individualized and appropriate recommendations are obtained and forwarded to the user. Alternatively, the recommendations can be forwarded to the user-designated caregivers, and/or health care providers for their reference.

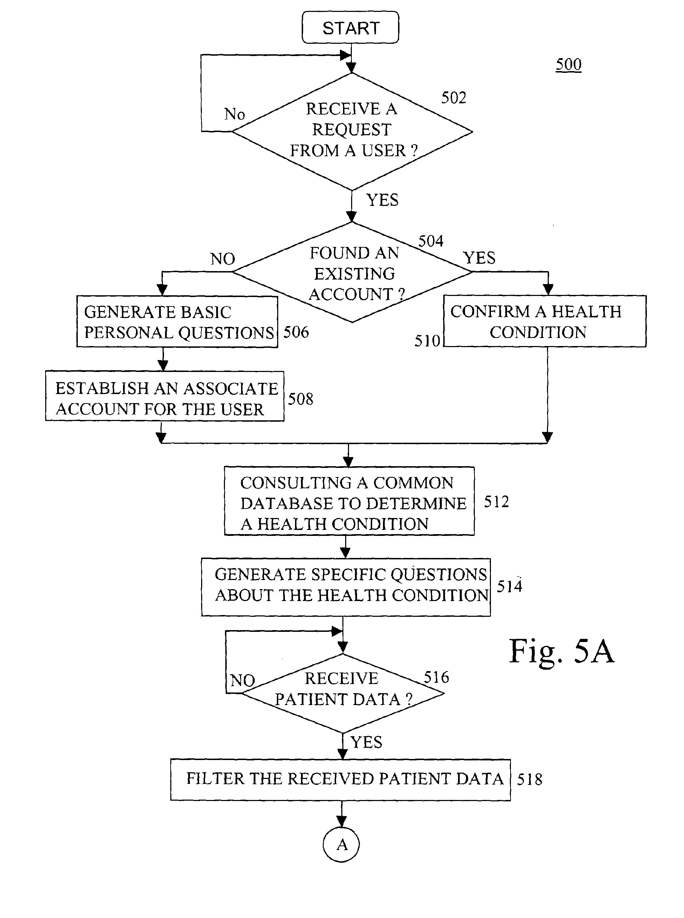

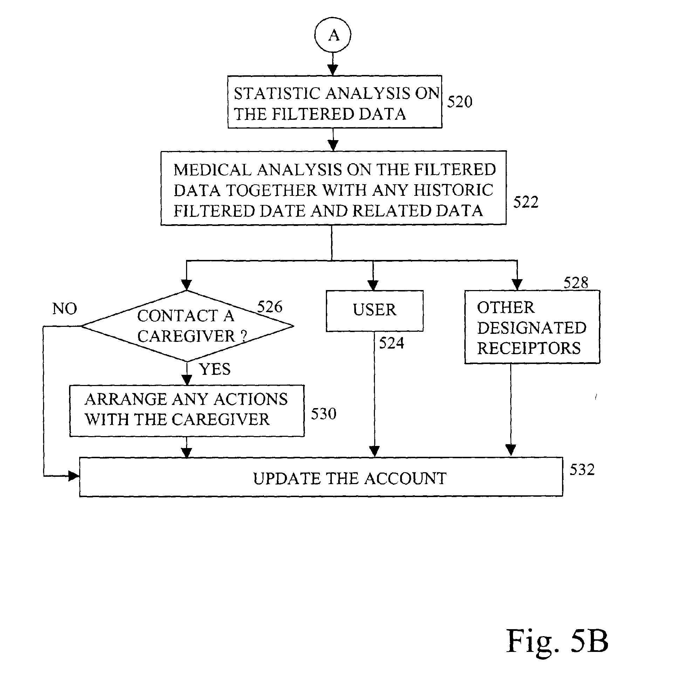

**Figure 5A** and **5B** show collectively a flow diagram **500** associated with an IHMT service process according to one embodiment and shall be understood in conjunction with the preceding figures. At **502,** the IHMT service process is awaiting a contact from a user. The contact is typically an IP request from a terminal device coupled to a network. Alternatively, the contact is an activation of a software module implementing the IHMT service process if the software module is resident in the terminal device. It is assumed in the following description that the IHMT service process is executing on a server coupled to the network.

When a request is received, a decision at **504** is to be made if the request is for an existing account or opening a new account. If the decision at **504** determines that the request is for a new account, flow diagram **500** goes to **506** to start a process of generating the new account. Initially, a set of personal questions are posted to the user. Examples of the personal questions may include username and password for the new account, age, gender and geographic location of the user as well as questions regarding medical history. At **508,** the account is established and typically maintained in an account database, such as the membership module **242a** of **Figure 2A.** At the same time, the account stores specific information on health condition(s) the user is concerned about.

When the decision at **504** is determined that that request is for an existing account, flow diagram **500** goes to **510** by retrieving the account and to confirm the health condition(s). As described above each account is preferably set up for the desired health condition(s) so that the appropriate IHMT module can be configured therefore. According to one embodiment, a question may be posted to the user to verify that the user is indeed consulting with the IHMT for an existing health condition. For example, the question may be asked, "Are you looking for advice on asthma you have been suffering". It should be noted that although an IHMT module is used for one specific health condition, several IHMT modules can be configured to interact with each other. As such a user may have several health conditions, so several IHMT modules for those conditions that are related can share data and analyses synergistically. For example, the diabetes IHMT module may extract analyses from the weight management IHMT module because weight management is an important aspect of diabetes management.

After flow diagram **500** determines what the user is looking for, flow diagram **500** moves to **512** where a common database is consulted. In one embodiment, the common database is an external knowledgebase **204** of **Figure 2B** that collects latest information about the specific health condition. At **514,** a set of specific questions regarding the health condition are assembled. Generally, the specific questions are configured to request various data be provided from the user. In some case, test data from a diagnostic test device may be required, in which case, the user has to get a test done and manages to have the test result supplied to the IHMT service. In other case, the history of the user from the associated account may be retrieved. Collectively, various data, regardless of their origins, regarding the specific health condition is referred to as patient data. At **516,** a decision is made to determine if the patient data is complete, usable or meaningful. If not, the user, the providers or other data resources will be notified before flow diagram 500 proceeds.

At **518,** the received or collected patient data is initially filtered to generated filtered data with respect to one or more knowledgebase to ensure that the filtered data to the subsequent medical analysis are usable. At **520,** a statistical analysis is performed on the data. In one embodiment, the statistical analysis is done among sets of patient data having similar or same health condition. The results from the statistical analysis can be useful or supportive to the subsequent medical analysis at **522.**

At **522,** the filtered patient data goes through an extensive medical analysis. As described above, the medical analysis is a process configured to simulate medical decision-making process and based on general medical decision making principles, common sense principles, and specific logic for the specific health condition. These principles may be derived from various sources such as standard medical practice guidelines, clinical research, mathematical relationships, biologic relationships, and consensus recommendations. In certain cases, the historic data of the user or similar health conditions are retrieved to make appropriate or customized decisions or recommendations for the user.

The recommendations are then provided to the user at **524** in various manners. In one embodiment, the recommendations include graphic representation in conjunction of texts. In another embodiment, the recommendations include tablet representation in conjunction of texts. In any case, the recommendations are customized and valid only for the user. According to one configuration, it is detected that the health condition of the user is beyond normal. According the recommendations include a request to send the results to designated recipients (e.g. private or family doctor, a parent if the user is minor). For example, if the user is found out that his/her recently glucose level is beyond normal and could be worsening if he/she continues his/her diet, the request is then asking the user if the recommendation shall be sent to a designated caregiver at **526.** When an approval from the user is received, flow diagram **500** goes on to arrange possible actions with the caregiver for the user at **530.** Depending on an exact implementation, the caregiver may be contacted or provide care procedures online or an appointment can be made with the caregiver. In certain circumstances, such as emergency situations as determined by the medical analysis engine or customizations made by the user or others, caregivers and/or other designated recipients can be automatically contacted and informed. In any event, the patient data about the user can be accessed by the caregiver or other designated recipients. At **532,** the user account is updated with the recommendations and follow-up procedures so that the user or other designated recipients can review or access the record anytime from anywhere.

The invention is preferably implemented in software or hardware or a combination of both. At least portions of the invention can also be embodied as computer readable code on a computer readable medium. The computer readable medium is any data storage device that can store data that can thereafter be read by a computer system.
Examples of the computer readable medium include read-only memory, random-access memory, disk drives, floppy disks, CD-ROMs, DVDs, magnetic tape, optical data storage devices, carrier waves. The computer readable media can also be distributed over network-coupled computer systems so that the computer readable code is stored and executed in a distributed fashion.

The advantages and benefits of the present invention are numerous. One of them is the mechanism provided by the present invention to provide online health care and wellbeing program. A user can get the needed services from anywhere at anytime. Another one is that the present invention provides customized medical recommendations based on extensive medical analysis of patient data provided by the user. Significantly different from some of existing online health services that provide recommendations based on samples collected from a group of users having similar health conditions, the recommendations provided to the user are through simulated medical decision-making process and based on general medical decision making principles, common sense principles, and specific logic for a specific health condition of the user. Still another one is that the various medical knowledge databases are used to support the medical decision-making process of the patient data about the specific health condition of the user so that reliable and customized recommendations can be generated. Other advantages and benefits have been made obvious through the detailed description herein and can be appreciated by those skilled in the art.

The present invention has been described in sufficient detail with a certain degree of particularity. It is understood to those skilled in the art that the present disclosure of embodiments has been made by way of examples only and that numerous changes in the arrangement and combination of parts may be resorted without departing from the spirit and scope of the invention as claimed. While the embodiments discussed herein may appear to include some limitations as to the presentation of the information units, in terms of the format and arrangement, the invention has applicability well beyond such embodiment, which can be appreciated by those skilled in the art. For example, the IHMT service has been largely described above to be provided through a server. In fact, the description above is equally applied to the case that the IHMT service is directly provided from a terminal device used by a user, in which case, the terminal device can be configured to communicate other servers to periodically update the various databases resident in the terminal device. Hence the IHMT module resident in the terminal device can make recommendations locally based on inputted data from the user thereof. Accordingly, the scope of the present invention is defined by the appended claims rather than the forgoing description of embodiments.

## Claims

1. A method for managing diseases and wellness online, the method comprising:
receiving patient data over a network from a user regarding a health condition;
filtering the patient data according to a first database to produce filtered patient data;
performing an analysis of the patient data; and
outputting, in response to the received patient data, a medical recommendation of the health condition based on a second database, wherein the medical recommendation includes what the user is suggested to do in regarding to the health condition.

2. The method of Claim 1, wherein the account lists the health condition about the user and wherein the first database includes a common knowledge database on health conditions, the knowledge database being constantly updated with other related servers on the network.

3. The method of Claim 1, wherein the receiving of the patient data comprises receiving diagnostic data from a diagnostic test device.

4. The method of Claim 1, wherein the analysis includes a statistical analysis and a medical analysis of the patient data.

5. A method for managing diseases and wellness online, the method comprising:
maintaining an account associated with a user having a health condition;
receiving over a network a request from the user to access the account;
composing a number of questions from the account after the user is authenticated;
receiving data from the user in response to the questions, wherein the data includes answers to the questions and/or diagnostic data received from a diagnostic test device pertaining to the health condition;
filtering the patient data according to a first database to produce filtered patient data, wherein the first database includes common knowledge database about the health condition and is being constantly updated with other related servers on the network;
performing an analysis of the patient data; and
providing to the user a medical recommendation of the health condition based on a second database, wherein the medical recommendation includes what the user is suggested to do in regarding to the health condition.

6. The method of Claims 1 or 5, wherein the second database is a medical management knowledgebase including static and/or dynamic information from multiple sources pertaining to the health condition.

7. The method of Claims 4 or 5, wherein the performing of the analysis of the patient data comprises:
obtaining statistic features of the patient data through the statistic analysis;
determining possible causes related to the health condition out of the patient data in conjunction with the statistic features.

8. The method of Claims 4 or 5, wherein the performing of the analysis of the patient data comprises, through the medical analysis, evaluating a state of the health condition using a medically related logic, risk stratification, and protocols/algorithms/guidelines that pertain to the health condition.

9. A machine-readable medium embodying instructions for execution by a processor, the instructions, when executed by the processor, causing the processor to produce structured documents, the machine-readable medium comprising:
program code for receiving patient data over a network from a user regarding a health condition;
program code for filtering the patient data according to a first database to produce filtered patient data;
program code for performing an analysis of the patient data; and
program code for outputting, in response to the received patient data, a medical recommendation of the health condition based on a second database, wherein the medical recommendation includes what the user is suggested to do in regarding to the health condition.

10. The machine-readable medium of Claim 9, wherein the account lists the health condition about the user and wherein the first database includes common knowledge database about the health condition, the knowledge database being constantly updated with other related servers on the network.

11. The machine-readable medium of Claim 9, wherein the program code for receiving the patient data comprises program code for receiving diagnostic data from a diagnostic test device.

12. The machine-readable medium of Claim 9, wherein the analysis includes a statistical analysis and a medical analysis of the patient data.
